# EUROPÄISCHE PATENTSCHRIFT

(11) **EP 1 943 194 B1**
(45) Veröffentlichungstag und Bekanntmachung des Hinweises auf die Patenterteilung: **12.12.2012**
(21) Anmeldenummer: 06806379.1
(22) Anmeldetag: 19.10.2006
(51) Int. Cl.: C02F 3/28, C12M 1/107

(54) **VERFAHREN UND REAKTOR ZUR ANAEROBEN BEHANDLUNG VON ABWASSER MITTELS EINES UASB**
PROCESS AND REACTOR FOR ANAEROBIC TREATMENT OF WASTEWATER BY MEANS OF A UASB
PROCEDE ET REACTEUR POUR TRAITEMENT ANAEROBIQUE D'EAUX USEES AU MOYEN D UN UASB

(30) Priorität: 25.10.2005 DE 102005050997
(43) Veröffentlichungstag der Anmeldung: 16.07.2008
(73) Patentinhaber: Aquatyx Wassertechnik GmbH, 88214 Ravensburg (DE)
(72) Erfinder: KNÖRLE, Ulrich, 88289 Waldburg (DE); WIDAK, Klaus, 88048 Friedrichshafen (DE)
(74) Vertreter: Zounek, Nikolai
(86) Internationale Anmeldenummer: PCT/EP2006/010066
(87) Internationale Veröffentlichungsnummer: WO 2007/048537

(56) Entgegenhaltungen:
- EP-A1- 0 808 805
- EP-A2- 0 629 584
- EP-A2- 1 291 326
- WO-A-02/02467
- WO-A-03/035560
- DE-A1- 2 421 935
- DE-A1- 10 314 933
- DE-A1- 10 358 234
- US-A- 4 707 252
- US-B1- 6 391 203

## Beschreibung

Die Erfindung betrifft ein Verfahren und einen Reaktor zur anaeroben Behandlung von Abwasser mittels eines anaeroben Aufström-Schlammbettes (Upflow Anaerobic Sludge Blanket = UASB) in einem Reaktor, durch Flotations-Trennung von Reaktorwasser, Biomasse und Biogas in einem ersten Abscheider, Trennung von Reaktorwasser und Biogas in einem zweiten Abscheider oberhalb des ersten Abscheiders, wobei ein Teil der als Schlamm oder Pelletschlämmung vorliegenden Biomasse im Kreislauf innerhalb des Reaktors geführt wird.

Das UASB-Verfahren wird bevorzugt eingesetzt zur Behandlung von Abwasser, das mit organischen Verunreinigungen belastet ist, wobei die Verunreinigungen in gelöster und ungelöster Form vorliegen. Das Anaerobic Sludge Blanket oder anaerobe Schlammbett enthält verschiedene Bakterienspezies, von denen einige zunächst die ungelösten organischen Verunreinigungen teilweise in wasserlösliche Stoffe - hauptsächlich organische Fettsäuren umsetzen und hydrolisieren. Hieran anschließend werden die gelösten Stoffe durch im Schlammbett enthaltene anaerobe Mikroorganismen in Biogas umgewandelt und so das Abwasser gereinigt. Biogas ist ein Gasgemisch aus den Bestandteilen Methan und Kohlendioxid sowie Schwefelwasserstoff und anderen Spurengasen. Wird nun vorausgesetzt, dass genügend Biomasse für den Abbau der im Wasser enthaltenen Stoffe verfügbar ist, ergibt sich die optimale hydraulische Aufenthaltszeit für das zu reinigende Wasser im Reaktor aus dem Verschmutzungsgrad, ausgedrückt beispielsweise als Chemischer Sauerstoffbedarf (CSB) und dem gewünschten Reinigungsgrad. Es ist bekannt, dass unter günstigen Bedingungen bereits bei hydraulischen Aufenthaltszeiten im Bereich von wenigen Stunden Reinigungsgrade von mehr als 90% erreichbar sind.

Derart hohe Leistungen lassen sich jedoch nur dann dauerhaft erzielen, wenn es gelingt, eine ausreichend große Menge an Biomasse dauerhaft im Reaktor zu halten oder gar einen Biomasse-Zuwachs zu erreichen. Die Wachstumsrate der anaeroben. Biomasse liegt im Bereich von ca. 0,05 • d⁻¹ und ist damit im Vergleich mit aerober Biomasse um ca. eine Größenordnung geringer. Es ist sicherzustellen, dass zumindest gleich viel Biomasse im Reaktor neu gebildet wird, wie ständig ausgeschwemmt wird. Im ungünstigsten Fall eines hohen hydraulischen Durchflusses mit einer niedrigen CSB-Konzentration ergibt sich ein erhöhtes Risiko für ein Ausschwemmen von Biomasse aus dem Reaktor, da die Bildungsrate für neue Biomasse auch abhängig vom zur Verfügung stehenden Substrat bzw. dem CSB des zugeführten Abwassers ist. Hieraus ist ersichtlich, dass der effektiven Biomasse-Rückhaltung eine entscheidende Bedeutung in Bezug auf die Leistungsfähigkeit eines anaeroben Reaktors zukommt.

In bekannten Anaerob-Reaktoren treten aufgrund der intensiven Produktion von Biogas hohe Aufströmgeschwindigkeiten von mehreren m/h auf. Sofern die Mikroorganismen nicht durch besondere Kultivierung auf speziellen Trägem fixiert sind, bewirkt die hohe Aufströmgeschwindigkeit eine Selektion von Spezies, die natürliche Aggregate bilden. Dieser Selektionsprozess beruht darauf, dass Spezies die keine Aggregate bilden, leichter sind, daher vermehrt aus dem Reaktor geschwemmt und schließlich von den aggregierenden Spezies verdrängt werden. Dieser Selektionsprozess erstreckt sich über Zeiträume von mehreren Monaten bis einigen Jahren und führt zur Bildung einer speziellen Schlammform, die gemeinhin als granulierter Schlamm oder auch "Pelletschlamm" bezeichnet wird. Diese "Pellets" haben eine Sinkgeschwindigkeit im Wasser von 50 bis 150 m/h, wohingegen Schlammflocken mit ca. 1 m/h absinken.

Typischerweise liegen die Schlarnmpellets als kugel- oder linsenförmiges Granulat mit Durchmessern von ca. 0,5 bis 2 mm vor. Das Granulat besteht in der Regel aus einem porösen Kalkgerüst, das im Laufe des Selektionsprozesses gebildet wird. Die Bakterien siedeln dabei nicht nur auf der Oberfläche des Granulats - wie beispielsweise bei einem massiven, geschlossenen Trägergranulat eines Festbett-Reaktors - sondern sind auch auf den inneren Oberflächen des Kalkgerüstes zu finden.

Durch die Tätigkeit der Mikroorganismen wird Biogas gebildet, das einerseits als Gasblasen aufsteigt, andererseits auch an der Biomasse anhaftet. Durch die teilweise Umhüllung der Pellets mit Biogas sinkt das spezifische Gewicht der Pellets unter die Dichte von Wasser und die Pellets steigen auf. Die nach oben treibenden Pellets werden durch in Abscheidern angeordnete Gashauben eingefangen und geben dort nach und nach das Gas wieder ab. Die Ablösung des Gases vom einzelnen Pellet wird durch den im Reaktor nach oben hin abnehmenden hydrostatischen Druck dadurch verstärkt, dass das Gas kompressibel ist und sich bei abnehmendem äußeren Druck ausdehnt. Die am Pellet anhaftenden Gasblasen werden mit abnehmendem Druck größer. Die aufgrund der Auftriebsbewegung im Wasser einwirkenden Reibungs- und Scherkräfte haben damit eine größere Angriffsfläche und die Ablösung der Gasblase vom Pellet wird begünstigt. Durch das Ablösen des Gases vom einzelnen Pellet steigt das spezifische Gewicht des Pellets wieder an, so dass dieses in den unteren Bereich des Reaktors zurücksinkt, wo der Prozess von Neuem beginnt. Durch die Gasbildung und -ablösung von Pellets kommt ein Kreislauf aus Flotation und Sedimentation in Gang.

Für die Umsetzung der organischen Verunreinigungen spielt der Stofftransport bzw. die Diffusion an der Oberfläche der Schlammpellets eine maßgebliche Rolle. Die Stärke des Diffusionsstromes eines bestimmten Stoffes ist proportional zu dessen Konzentrationsgefälle vom Abwasser zu den Mikroorganismen im Schlammpellet. Letzteres ist teilweise von einer Hülle aus anhaftendem Biogas umgeben. Das Konzentrationsgefälle und die Diffusion sind umgekehrt proportional zur Dicke dieser anhaftenden Gashülle. Der Umsatz von organischen Verbindungen und damit verbunden die Effizienz des Reinigungsverfahrens lässt sich somit steigern, indem die den Schlammpellets anhaftende Hülle aus Biogas möglichst schnell abgelöst wird. Es ist hinlänglich bekannt, dass die den Schlammpellets anhaftende Gashülle durch hohe Turbulenz, d.h. durch große Geschwindigkeitsgradienten verringert wird. Allerdings ist hierbei zu berücksichtigen, dass zu heftige Umwälzungen im Reaktor und die damit verbundenen mechanischen Scherkräfte den Wachsturnsprozess der Schlammpellets nachhaltig stören oder verhindern können. Im Extremfall kann das fragile Granulat sogar zerstört werden. Dementsprechend ist eine effektive Umwälzung bzw. Kreislaufführung der Biomasse mit schonender Gasabscheidung erstrebenswert.

EP 0 170 332 A1 offenbart ein Verfahren und eine Vorrichtung zur anaeroben Behandlung von Abwasser mittels UASB, bei dem ein Behälter verwendet wird, in dessen unteren Bereich das zu reinigende Abwasser geleitet und aus dessen oberen Bereich das gereinigte Abwasser abgeleitet wird. Im Behälter sind anaerobe Mikroorganismen tätig. Zwischen dem Abwassereinlass und dem Überlauf für das gereinigte Abwasser befinden sich im Behälter übereinandergestapelte Gassammler in Form von Hauben, deren oberer Bereich über eine Leitung mit einer Gas-Schlamm-Trenneinrichtung verbunden ist. Durch die Tätigkeit der Mikroorganismen wird Gas erzeugt, das sich am Schlamm anlagert, so dass dieser als sogenannter Schwimmschlamm nach oben aufschwimmt. Dieser Schwimmschlamm wird durch die Haube eingefangen und gibt nach und nach sein Gas wieder ab, so dass er wieder schwerer wird und als sogenannter Sinkschlamm zurück auf den Boden sinkt. Das von den Pellets abgegebene Gas steigt zusammen mit den von den Hauben eingefangenen, freien Gasblasen weiter in den Leitungen nach oben und reißt dabei Schwimmschlammpartikel und Flüssigkeit mit, die in der Gas-Schlamm-Trennkammer abgetrennt werden. Das Gas wird zweckmäßigerweise abgeführt, während die mitgerissene Flüssigkeit, die auch Schlammpartikel enthalten kann, in eine Falleitung gelangt, die zurück auf den Boden des Behälters führt. Dadurch soll der Sinkschlamm am Boden aufgewirbelt werden, was zu einer Auflockerung der Schlammzone im Bodenbereich und einer besseren Vermischung der Mikroorganismen mit dem ankommenden Abwasser führen soll. Da Wasser jedoch relativ schwer ist, ist die Menge des durch das aufschwimmende Gas transportierbaren Abwassers und somit auch die Verwirbelungsleistung des rückgeführten Abwassers begrenzt. Weiterhin ist bekannt, dass Abwasserreaktoren dieser Art über Reaktorhöhen von mindestens 11 m verfügen müssen, bevor der dort beschriebene Effekt eintritt.

EP 0 244 029 A1 beschreibt einen UASB-Reaktor der mit einer Vorrichtung zur Trennung der drei Phasen Wasser, Schlamm und Biogas ausgestattet ist. Die Trennvorrichtung umfasst Gashauben, die über Durchtrittsöffnungen mit einem Gassammelkasten verbunden sind, wobei die Durchtrittsöffnungen im oberen Bereich der Gashauben unterhalb des Haubenfirstes angeordnet sind. Zusätzlich ist jede Gashaube im Inneren mit Rückhaltekästen ausgerüstet. Die Rückhaltekästen und die Durchtrittsöffnung sind so gestaltet, dass sich ein Gaspolster ausbildet, welches als Barriere für Wasser und Schlamm fungiert.

WO 99/51532 lehrt ein Verfahren und eine Vorrichtung zur anaeroben Reinigung von Abwasser in einem Abwasser und Schlamm aufnehmenden Behälter unter Gasentwicklung. Das sich entwickelnde Gas wird durch einen Gassammler aufgefangen und der durch das aufsteigende Gas angetriebene Kreislauf wird zum Auflockern des auf den Boden des Behälters abgesunkenen Sinkschlamms verwendet. Durch einen Gashebeeffekt des aufsteigenden Gases wird der Sinkschlamm vom Boden abgesaugt und getrennt vom Abwasser in den oberen Bereich des Behälters und zurück in das Abwasser geleitet.

Aus der DE 199 31 085 A1 ist ein UASB-Verfahren zur anaeroben Reinigung von Abwasser bekannt, bei dem das gebildete Biogas und der gasbeladene Schwimmschlamm in Gashauben aufgefangen werden. Das Gas bildet unterhalb eines Dachfirstes der Gashauben einen Gaspolster und sammelt sich oberhalb des Schwimmschlammes. Das Gas gelangt über eine Leitung in eine U-förmige Rohrkrümmung, in der sich ein Gaspolster ausbildet, der das Aufsteigen und Mitreißen von Schwimmschlamm in eine weitere Leitung verhindert, die mit einer Steigleitung verbunden ist. Dadurch gelangt allein Gas über die weitere Leitung in die Steigleitung. In dieser verursacht das Gas eine starke, aufsteigende Strömung und dadurch einen Unterdruck an der Ansaugöffnung der Steigleitung bzw. Ansaugleitung im oberen Bereich des Reaktorbehälters. Die Ansaugöffnung befindet sich unterhalb eines Überlaufes für das gereinigte Abwasser, d. h. knapp unterhalb des Wasserspiegels und somit oberhalb der beiden Sammelsysteme für Biogas und Schwimmschlamm. Durch den Unterdruck an der Ansaugöffnung wird Medium aus dem oberen Bereich des Reaktorbehälters, d. h. oberhalb der beiden Sammelsysteme angesaugt. Das Medium ist dabei weitgehend gereinigtes Wasser mit Schwimmschlammresten.

EP 0 711 732 A2 beschreibt ein Modul für einen Reaktor zur anaeroben Reinigung von Abwasser, enthaltend eine den Wasserstand im Modul festlegende obere Überlaufschwelle für das gereinigte Abwasser, mehrere über den ganzen Modulquerschnitt gestaffelt angeordnete Auffanghauben für Biogas mit einer Ableitung in einen Gassammelraum und eine obere Abzugsleitung für die von den Abzugshauben nicht erfasste Abluft. Oberhalb des jeweiligen Abscheiders wird die biogasreiche Luft in einen Gassammelraum geführt. Die Entnahme des Biogases aus den Auffanghauben erfolgt über eine kurze Rohrleitung.

EP-A2-1 291 326 beschreibt einen Dreiphasen-Separator zum Abtrennen von Gas und Teilchen mit mindestens zwei Gassammelhauben, die sich in horizontaler Richtung erstrecken und jeweils einen Plattenabschnitt aufweisen, wobei die Plattenabschnitte in Abwärtsrichtung konvergieren und an ihrem unteren Ende mindestens einen Durchgang für Teilchen und Wasser freilassen. DE 103 58 234 A und DE 103 14 933 A betreffen ein Verfahren und einen Reaktor zum anaeroben Reinigen von Abwasser nach dem UASB-Prinzip, bei dem Aktiv-Pellets weitgehend zurückgehalten und überschüssige Pellets in einen Gassammelraum geleitet und von dort in den Bodenbereich zurückgeführt werden. Der Reaktor weist zwei übereinander angeordnete Haubensysteme auf, die jeweils einen Gassammelkasten mit mehreren Gassammelhauben umfassen. Jede der Gassammelhauben ist über einen Gasüberleitungskasten mit einem der Gassammelkästen verbunden. Die Wand des Gassammelkastens hat eine Durchlassöffnung für Gas, die unterhalb des Gasüberleitungskasten und oberhalb der Gassammelhaube und deren First angeordnet ist. Um von der Gassammelhaube in den Gassammelkasten zu gelangen, müssen Pellets innerhalb des Gasüberleitungskastens eine aufwärts entgegen der Schwerkraft schwimmen. Die Anordnung gemäß DE 103 58 234 A und DE 103 14 933 A verkörpert das Prinzip eines umgedrehten Syphons und verhindert weitgehend, dass Pellets von der Gassammelhaube durch den Gasüberleitungskasten in den Gassammelkasten übertreten. Darüber hinaus offenbart DE 103 58 234 A Gashauben, die mit mehreren nach Art einer Labyrinthdichtung angeordneten Trennwänden ausgestattet sind. Die Trennwände hindern Pellets daran, von der Gassammelhaube in den Gasüberleitungskasten überzutreten.

Aus US-A-4 707 252 ist ein Reaktor mit Wirbelbett aus Granulatmaterial bekannt, in dem die aerob und anaerob zu behandelnde Flüssigkeit von unten nach oben durch das Wirbelbett strömt. Das Gas für die aerob zu behandelnde Flüssigkeit ist Luft, Sauerstoff oder mit Sauerstoff angereicherte Luft. Das Gas für die anaerob zu behandelnde Flüssigkeit wird im Wirbelbett selbst erzeugt. Der Reaktor besteht aus drei miteinander in Verbindung stehenden Abschnitten, nämlich einer Fluidisierungszone, einer Entgasungskammer und einer Trenn- und Dekantierkammer. In der Fluidisierungszone wird Gas der zu behandelnden Flüssigkeit zugesetzt, die das Wirbelbett durchströmt, wodurch eine Dreiphasenmischung aus Gas-Flüssigkeit-Granulatmaterial entsteht. Diese Mischung strömt über ein Rohr in die Entgasungskammer, in der überschüssiges Gas von dem Granulatmaterial abgetrennt und über eine Ablaufleitung abgeführt wird. Die verbleibende Flüssigkeit-Granulat-Mischung strömt in die Trenn- und Dekantierkammer, in der das Granulat von der Flüssigkeit getrennt und über ein Recyclingrohr in das Wirbelbett zurück geführt wird. Die behandelte Flüssigkeit strömt über einen Kanal aus dem Reaktor heraus.

EP-A1-0 808 805 offenbart ein Verfahren und einen Schlammbettreaktor zum anaeroben Reinigen von Abwasser, bei dem aufgrund besonderer Strömungsverhältnisse Abwasser und Biomasse verstärkt vermischt werden. Der Reaktor umfasst ein Dreiphasentrennsystem mit Gasabscheideelemente, die innerhalb des Schlammbettes angeordnet sind. Mittels des Dreiphasentrennsystems wird die Biomasse zurückgehalten, während andererseits behandeltes Wasser und bei der Vergärung gebildetes Biogas separat abgezogen werden.

WO 02/02467 A betrifft eine Vorrichtung zur Dreiphasentrennung bei der Behandlung von Abwasser und Schlamm mit einem in einem Absetzbecken angeordneten Gassammler zum Auffangen des bei dem Umwandlungsprozeß in der Vorrichtung entstehenden oder in diese eingeleiteten Gases. Der Gassammler weist eine gegenüber der Horizontalen geneigte und das Abgleiten von Klärschlamm ermöglichende Außenfläche mit einstellbarem Neigungswinkel auf.

Aus der EP-A2-0 629 584 ist ein Biogasreaktor des UASB-Typs mit Auffanghauben für Biogas bekannt. Die Auffanghauben weisen in einem vorgegebenen vertikalen Höhenbereich einen konstanten horizontalen Querschnitt auf. Der Reaktor wird mittels Gasgegendruck so betrieben, dass der Flüssigkeitsspiegel in dem vorgegebenen vertikalen Höhenbereich der Auffanghauben schwankt.

Untersuchungen haben gezeigt, dass lediglich 10 bis 20 % der im Reaktor vorhandenen Biomasse aktiv am Reinigungsprozess teilnimmt. 80 bis 90 % der vorhandenen Biomasse liefert praktisch keinen Beitrag zur Reinigung des Abwassers. Dementsprechend kommt der Rückführung von Biomasse und der Beteiligung eines größeren Anteils Biomasse aus dem Schlammbett sowie der schonenden Abscheidung von Biogas eine entscheidende Bedeutung für die Leistungsfähigkeit des UASB-Verfahrens zu.

Die bekannten Verfahren offenbaren keine Methode zur effektiven und schonenden Rückführung von Biomasse. Aufgrund ihrer Konstruktion mit verwinkelten und engen Leitungen begünstigen die bekannten Vorrichtungen Verstopfungen durch mitgeführten Schlamm. Im Fall einer Verstopfung sind die kritischen Anlagenteile von außen praktisch nur mit Spezialwerkzeugen, wie z.B. gekrümmten oder flexiblen Schiebern zugänglich, was Reinigung und Wartung erheblich erschwert. Darüber hinaus ist der Kreislauf bzw. Durchsatz an Biomasse niedrig und zu wenig frische Biomasse aus dem Schlammbett wirkt aktiv an der Abwasserreinigung mit.

Die Aufgabe der vorliegenden Erfindung besteht darin, ein Verfahren und einen Reaktor zur anaeroben Behandlung von Abwasser bereitzustellen, die gegenüber dem Stand der Technik erhöhte Effizienz und Durchsatz aufweisen und bei denen ein größerer Anteil der im Schlammbett vorhandenen Biomasse aktiv an der Reinigung des Abwassers beteiligt wird und eine teilweise Vermischung von Biogas und Biomasse vor dem Eintritt in eine Steigleitung zu einem Gasseparator unter Vermeidung von Verstopfungen der Steigleitung erfolgt.

Diese Aufgabe wird gelöst durch ein Verfahren zur anaeroben Behandlung von Abwasser mittels eines anaeroben Aufström-Schlammbettes (Upflow Anaerobic Sludge Blanket = UASB), bei dem eine als Schlamm oder Pelletschlämmung vorliegende Biomasse in einem Kreislauf geführt wird, das durch folgende Verfahrensschritte gekennzeichnet ist:
(a) Vermischung von Biogas und Biomasse zu einem Biogemenge nach dem Austritt aus dem ersten Abscheider,
(b) Förderung des Biogemenges und des im zweiten Abscheider abgetrennten Biogases zu einem Gasseparator,
(c) Abscheidung des Biogases, das im Verfahrensschritt (b) anfällt, im Gasseparator unter Freisetzung von Biomasse,
(d) Rückführung der freigesetzten Biomasse bis unterhalb des Schlammbettes UASB.

Bei dem erfindungsgemäßen Verfahren ist der Anteil von rückgeführter zu gesamter Biomasse im Reaktor pro Tag größer 0, 1 • d⁻¹, insbesondere größer 2 • d⁻¹ und besonders bevorzugt größer 10 • d⁻¹.

Ferner hat die vorliegende Erfindung die Aufgabe, einen Reaktor zur anaeroben Behandlung von Abwasser mittels eines anaeroben Aufström-Schlammbettes (Upflow Anaerobic Sludge Blanket = UASB) zu schaffen, der es ermöglicht, Biomasse in einem Kreislauf zu führen. Weiterer Gegenstand der Erfindung ist somit ein Reaktor zur Durchführung des erfindungsgemäßen Verfahrens. Dieser Reaktor umfasst einen Reaktortank, Leitungen, einen Abwassermischer, einen ersten und mindestens einen zweiten Abscheider zur Flotations-Trennung von Reaktorwasser, Biomasse und Biogas, einen oder mehrere Mischer zur Vermischung von Biomasse und Biogas und einen Gasseparator zur Trennung von Biomasse und Biogas, wobei der zweite Abscheider vertikal über dem ersten Abscheider angeordnet ist, jeder Abscheider eine oder mehrere Gashauben aufweist, und die Gashauben des ersten Abscheiders mit einem oder mehreren Mischern zur Vermischung von Biomasse und Biogas zu einem Biogemenge verbunden sind und die Mischer über eine Leitung an einen Gasseparator zur Trennung von Biomasse und Biogas angeschlossen sind.

Neben hoher Effizienz und Durchsatz ermöglicht die Erfindung eine einfache und kostengünstige Konstruktion eines Reaktors mit geringer Verstopfungsneigung, direktem Zugang zu kritischen Anlagenteilen und damit verbunden verringertem Wartungsaufwand.

Die Erfindung wird im folgenden anhand von Ausführungsbeispielen und unter Bezugnahme auf schematische Zeichnungen näher beschrieben. Es zeigen:
- Fig. 1: einen Reaktor mit Biomasse-Kreislauf
- Fig. 2: Gashauben eines ersten und eines weiteren Abscheiders
- Fig. 3a - 3b: Gashauben mit gemeinsamem Mischer
- Fig. 3c - 3d: Gashauben mit separaten Mischern
- Fig. 4a - 4d: Vermischung von Biogas und Biomasse in einem Mischer
- Fig. 5a - 5d: verschiedene erfindungsgemäße Mischer
- Fig. 6a - 6b: Mischer mit Rückhaltekasten und Wartungsöffnung
- Fig. 7: Abscheider mit übereinander angeordneten Gashauben
- Fig. 8: Mischer mit zwei Einlasskanälen

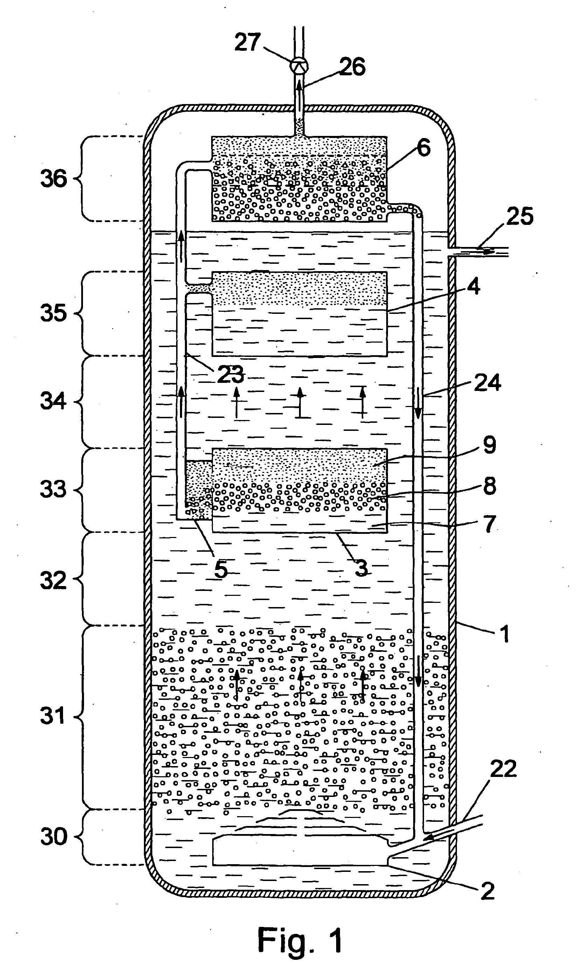

Fig. 1 zeigt schematisch einen erfindungsgemäßen Reaktor, umfassend einen Reaktortank 1, Leitungen 22 bis 26, einen Abwassermischer 2, einen ersten und einen zweiten Abscheider 33, 35 zur Trennung von Reaktorwasser 7, Biomasse 8 und Biogas 9, einen Mischer 5 zur Vermischung von Biomasse 8 und Biogas 9 und einen Gasseparator 6 zur Trennung von Biomasse 8 und Biogas 9. Der zweite Abscheider 35 ist vertikal über dem ersten Abscheider 33 angeordnet. Jeder Abscheider 33,35 weist mindestens eine Gashaube 3, 4 auf, wobei die Gashaube(n) 3 des ersten Abscheiders 33 mit dem Mischer 5 verbunden ist/sind. Über die Zufuhrleitung 22 wird dem Reaktor organisch belastetes Abwasser zugeführt und von dem in einer Bodenzone 30 angeordneten Abwassermischer 2 unter leichter Wirbelbildung in eine Fermentationszone 31 abgegeben. Gereinigtes Abwasser wird über die im oberen Bereich des Reaktors angeordnete Abflussleitung 25 abgeführt. In der Fermentationszone 31 befindet sich die Biomasse 8 als Bett aus Schlamm oder Granulatschlämmung. Die in der Biomasse 8 enthaltenen Bakterien bauen die organischen Inhaltsstoffe des Abwassers ab, wobei Biogas 9 gebildet wird. In der Fermentationszone 31 geht das Biogas 9 teilweise in Lösung in das Reaktorwasser 7 über, zum anderen Teil bildet es feine Bläschen die an der Biomasse 8 haften oder frei im Reaktorwasser 7 aufsteigen. Die an der Bildung von Biogas 9 beteiligte Biomasse 8 wird durch das anhaftende Biogas 9 leichter als das Reaktorwasser 7 und beginnt ebenso wie die freien Gasbläschen aus der Fermentationszone 31 in eine erste Driftzone 32 aufzusteigen. Das von der Biomasse 8 erzeugte Biogas 9 verursacht eine aufwärts gerichtete Strömung sowohl von Biomasse 8 wie auch von Reaktorwasser 7.

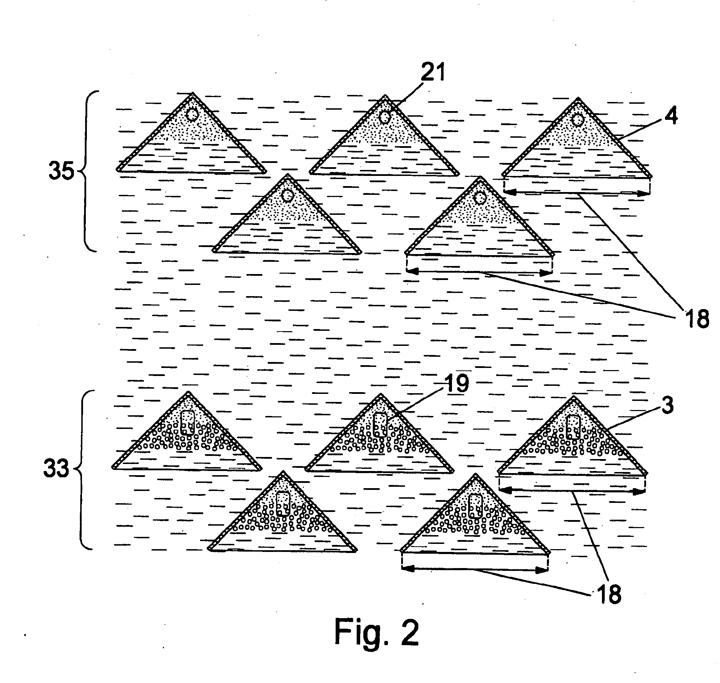

Oberhalb der ersten Driftzone 32 ist der erste Abscheider 33 mit einer oder mehreren Gashauben 3 (siehe Fig. 2) angeordnet. Die freien Bläschen aus Biogas 9 fangen sich in den Gashauben 3 und bilden ein Gaspolster. Direkt unterhalb des Gaspolsters bildet sich eine Flotationsschicht bestehend aus mit Biogas 9 durchsetzter Biomasse 8. Aus den Gashauben 3 strömen Biomasse 8 und Biogas 9 in den Mischer 5. Der Mischer 5 weist eine besondere Gestalt auf, die in Verbindung mit den hydrostatischen Druckverhältnissen und der Strömungsdynamik im Mischer 5 bzw. in der mit dem Mischer 5 verbundenen Förderleitung 23 eine intensive Vermischung und/oder Verwirbelung von Biomasse 8 und Biogas 9 zu einem Biogemenge bewirkt. Vom Mischer 5 wird das Biogemenge über die Förderleitung 23 in den Gasseparator 6 gefördert. Wie zuvor die Vermischung beruht die Förderung des Biogemenges auf den hydrostatischen und strömungsdynamischen Verhältnissen, die im folgenden kurz erörtert werden. In den Gashauben 3 herrscht ein Druck, der sich aus der Summe der über dem Abscheider 33 lastenden hydrostatischen Säule aus Reaktorwasser 7 und dem Umgebungsdruck von 1 atm ergibt und der je nach Füllhöhe des Reaktors bis zu 2 atm beträgt. Demgegenüber fällt der Druck in der Förderleitung 23 stetig ab auf den Druckwert in dem mit Förderleitung 23 verbundenen Abscheider 35. Der Abscheider 35 ist nahe der Oberfläche der Wassersäule angeordnet, so dass der dort herrschende Gesamtdruck nur geringfügig höher als der Umgebungsdruck von 1 atm ist. Vom Abscheider 35 zum Gasseparator 6 fällt der Druck gegebenenfalls weiter auf 1 atm ab. In einer besonderen Ausführungsform der Erfindung ist der Druck im Gasseparator 6 über ein Durchflussregelventil 27 in der Abströmleitung 26 steuerbar. Hierbei dient das Durchflussregelventil 27 primär zur Steuerung des Volumens des abfließenden Biogases 9 und damit zur Steuerung der Dicke der Gaspolster in den Gashauben 3, 4.

Die Förderung in der Förderleitung 23 basiert auf dem Prinzip der kommunizierenden Röhren, wobei die Flüssigkeitssäule im Reaktor über dem Abscheider 33 dem ersten Arm eines U-förmigen Rohres, die mit Biogemenge und Biogas 9 gefüllte Förderleitung 23 dem zweiten Arm und die Gashauben 3 mit den Mischern 5 dem gekrümmten Teil des U entsprechen. Die Förderleitung 23 verläuft im Reaktortank 1, wie Fig. 1 zeigt. Ebenso ist es vorgesehen, dass die Förderleitung 23 teilweise oder vollständig außerhalb des Reaktortanks 1 angeordnet ist.

Neben dem Prinzip der kommunizierenden Röhren kann auch die strömungsdynamische Mitführung des Biogemenges durch schnell strömendes Biogas 9 genutzt werden. Ein hohe Gasbildungsrate im Reaktor in Verbindung mit kleinem Querschnitt der Förderleitung 23 bewirkt hohe Geschwindigkeit und Turbulenz im abströmenden Biogas 9, wodurch das Biogemenge in dem Mischer 5 und in der Förderleitung 23 mitgerissen wird. Um die strömungsdynamische Förderung des Biogemenges zu verstärken, ist es vorteilhaft, den Querschnitt der Förderleitung 23 um einen Faktor 1 : 60 bis 1 : 400 kleiner als den Querschnitt des Reaktortanks zu dimensionieren. Die Mischer 5 und die Förderleitung 23 befinden sich bevorzugt im Reaktortank 1, können jedoch ebenso zum Teil oder vollständig außerhalb des Reaktortanks 1 angeordnet sein.

Der Großteil der mit Biogas 9 durchsetzten flotierenden Biomasse 8 wird im Abscheider 33 aufgefangen und zum Gasseparator 6 gefördert. Das Reaktorwasser 7, das durch den Abscheider 33 hindurch in eine zweite Driftzone 34 gelangt, führt nur eine geringe Menge flotierender Biomasse 8 mit. In der Driftzone 34 nimmt der hydrostatische Druck stetig bis auf 1 atm ab. Hierdurch bildet das der flotierenden Biomasse 8 anhaftende Biogas 9 zunehmend größere Blasen, die sich schließlich ablösen. Durch die Ablösung des Biogases 9 nimmt das spezifische Gewicht der Biomasse 8 wieder zu, so dass diese auf den Boden des Reaktors zurücksinkt. Die Abschirmung durch den ersten Abscheider 33 in Verbindung mit der erhöhten Gasabgabe in der Driftzone 34 bewirkt, dass das Reaktorwasser 7, das an die Oberfläche der Wassersäule im Reaktor gelangt und über die Abflussleitung 25 abgeführt wird, praktisch frei von Biomasse 8 ist. Das in den Gashauben 4 gesammelte Biogas 9 strömt über die Förderleitung 23 in den Gasseparator 6. Die Gashauben 4 sind in einem geringen Abstand unterhalb der Oberfläche der Wassersäule im Reaktor angeordnet, so dass der hydrostatische Druck geringfügig höher als 1 atm ist, wodurch das Biogas 9 aus den Gashauben 4 zum Gasseparator 6 gefördert wird.

Im Gasseparator 6 werden die im Biogemenge enthaltene Biomasse 8 und das Biogas 9 getrennt. Aus dem Gasseparator 6 fließt die Biomasse 8 unter Einwirkung der Schwerkraft über die Rücklaufleitung 24 in den Reaktor zurück. In einer bevorzugten Ausführung der Erfindung ist die Rücklaufleitung 24 mit der Zufuhrleitung 22 verbunden, so dass die rückgeführte Biomasse 8 mit zufließendem Abwasser vermischt wird.

Fig. 2 zeigt einen Schnitt durch die Abscheider 33, 35 mit den Gashauben 3, 4. Bevorzugt sind die Gashauben 3, 4 Hohlkörper mit einer polygonalen oder halbkreisartig gekrümmten Hüllwand in Form eines umgekehrten V oder umgekehrten U mit einer nach unten weisenden Öffnung 18. Jede der Gashauben 3 ist durch ebene Stirnwände begrenzt, wobei mindestens eine Stirnwand eine oder mehrere Durchtrittsöffnungen 19 für Biomasse 8 und Biogas 9 aufweist. In einer alternativen Ausführungsform der Erfindung bilden die Mischer 5 mindestens eine der Stirnwände der Gashaube 3. In Ausgestaltung der Erfindung sind die Durchtrittsöffnungen (19) im oberen Bereich der Stimwand unterhalb des Firstes der Gashauben (3) angeordnet. Ähnlich zu den Gashauben 3 sind auch die Gashauben 4 durch ebene Stimwände begrenzt, wobei mindestens eine Stirnwand Durchtrittsöfnungen 21 für Biogas 9 aufweist. Das in den Gashauben 4 gesammelte Biogas 9 gelangt über die Durchtrittsöffnungen 21 und die Förderleitung 23 in den Gasseparator 6. Die Durchtrittsöffnungen 21 sind mittels separater Verbindungsleitungen oder mittels Sammelleitungen an die Förderleitung 23 angeschlossen. In weiterer Ausgestaltung der Erfindung sind die Durchtrittsöffnungen (21) im oberen Bereich der Stirnwand unterhalb des Firstes der Gashauben (4) angeordnet. Wie in Fig. 2 dargestellt, sind die Gashauben 3, 4 in jedem der Abscheider 33, 35 in zwei oder mehreren übereinander liegenden horizontalen Ebenen angeordnet. In einer Ebene sind die Gashauben 3, 4 jeweils parallel und voneinander beabstandet angeordnet. Durch die Spalte zwischen benachbarten Gashauben 3, 4 tritt aufsteigendes Reaktorwasser 7 hindurch und strömt nach oben. In jedem der Abscheider 33, 35 sind die Reihen der Gashauben 3, 4 in übereinander liegenden Ebenen derart zueinander versetzt, dass die vertikalen Projektionen der Öffnungen 18 der Gashauben 3, 4 eine geschlossene Fläche bilden, die den Innenquerschnitt des Reaktortanks 1 teilweise oder vollständig abdeckt. Durch diese labyrinthartige Anordnung der Gashauben 3, 4 werden Biomasse 8 und Biogas 9 praktisch vollständig aufgefangen.

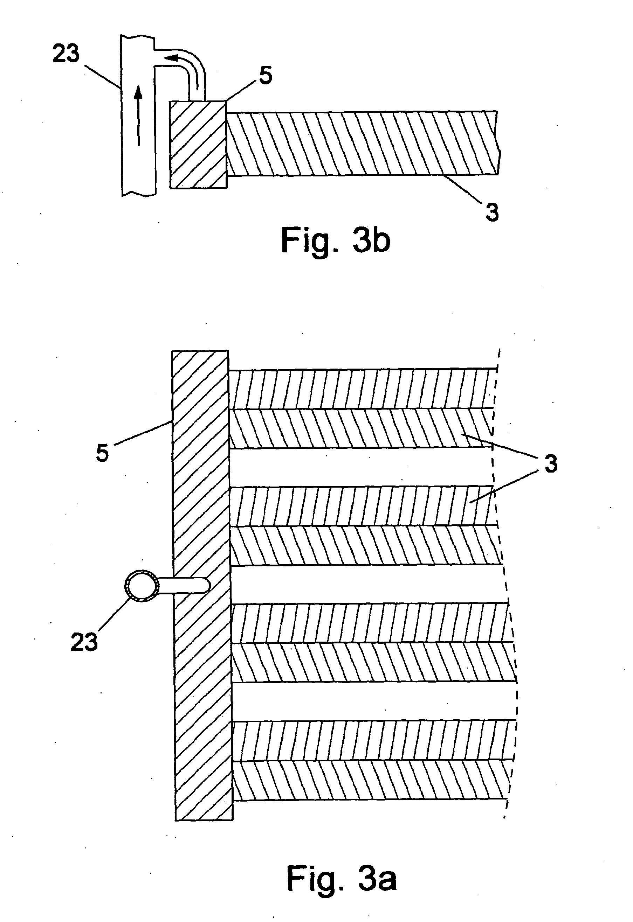

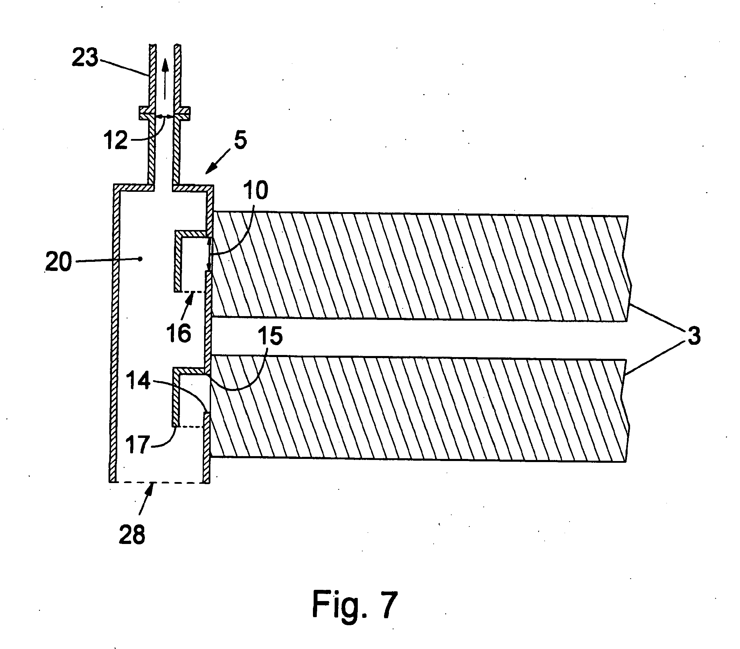

Wie in Fig. 3a in Draufsicht und in Fig. 3b in Seitenansicht gezeigt, umfasst die Erfindung eine Ausführungsform mit einem ersten Abscheider, bei dem mehrere - z.B. in einer horizontalen Ebene angeordnete- Gashauben 3 mit dem Mischer 5 verbunden sind. Des weiteren können auch die in zwei oder mehreren horizontalen Ebenen übereinander angeordneten Gashauben 3 mit einem gemeinsamen Mischer 5 verbunden sein (siehe Fig. 7).

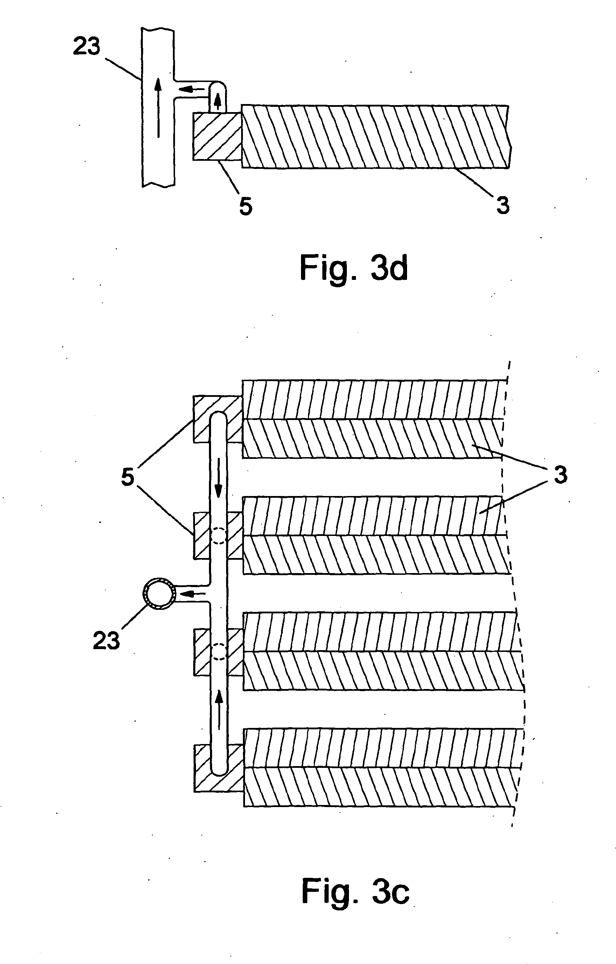

Eine alternative, in den Fig. 3c und 3d in Draufsicht und Seitenansicht dargestellte Ausführungsform der Erfindung betrifft einen ersten Abscheider bei dem die Gashauben 3 jeweils mit separaten Mischern 5 verbunden sind. In dieser Ausführungsform sind die Mischer 5 z.B. über eine Sammelleitung oder über separate Verbindungsleitungen an die Förderleitung 23 angeschlossen.

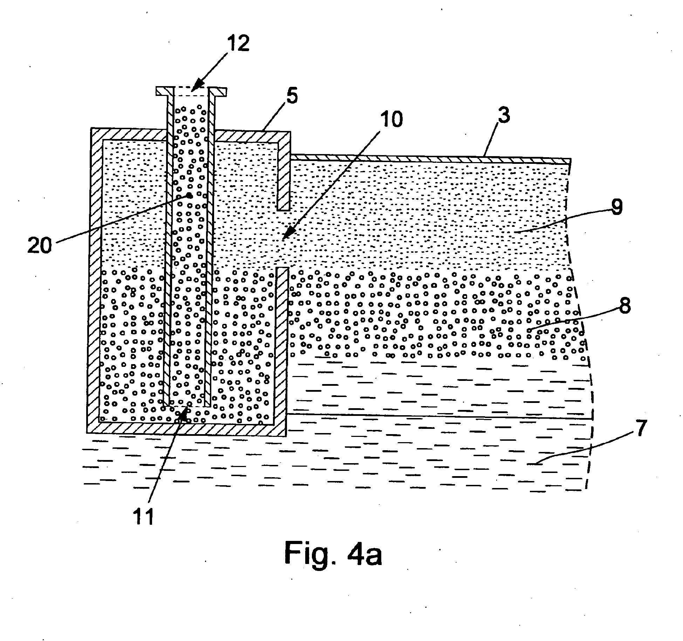

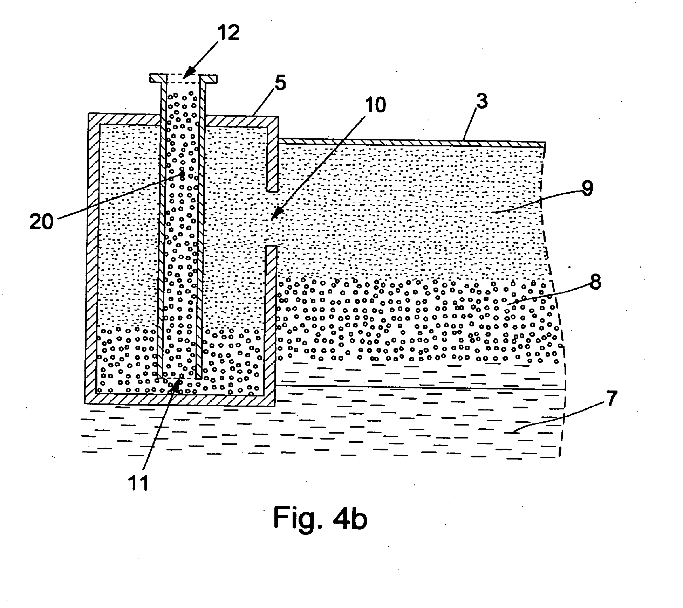

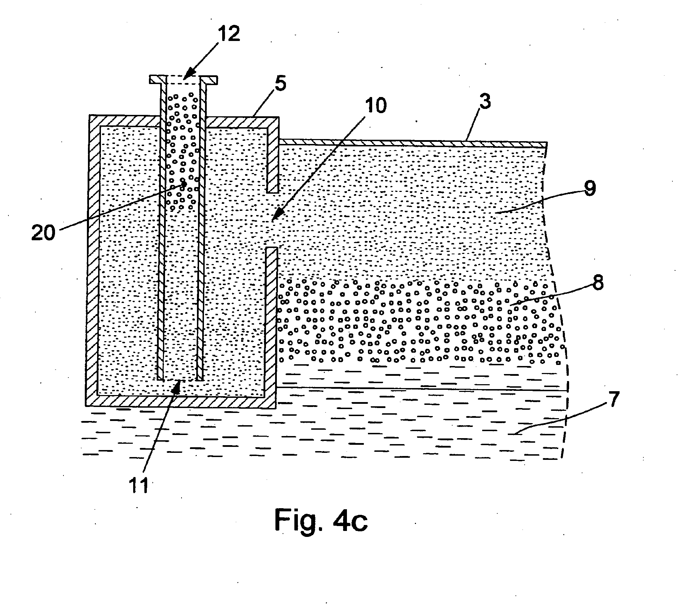

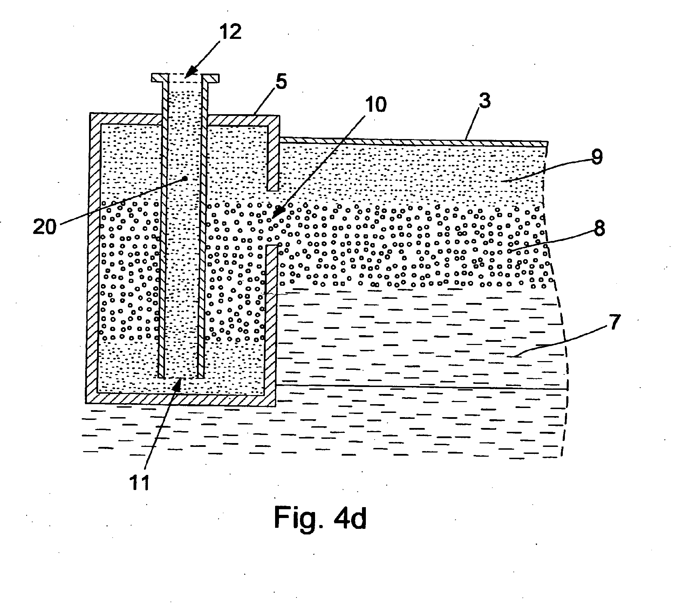

Die Fig. 4a bis 4d illustrieren die Funktionsweise der Mischer 5. Die Mischer 5 sind Hohlkörper mit einem oder mehreren Einlässen 10 für Biomasse 8 und Biogas 9, einer oder mehreren Durchführungen 11 und einem oder mehreren Kanälen 20 sowie einer oder mehreren Auslassöffnungen 12, die über die Förderleitung 23 mit dem Gasseparator 6 verbunden sind. In dem in den Fig. 4a bis 4d gezeigten Beispiel bildet der Mischer 5 eine stirnseitige Begrenzung der Gashauben 3. Das in den Gashauben 3 aufgefangene Biogas 9 und die darunter liegende Schicht aus flotierender Biomasse 8 strömen durch die Einlässe 10 in den Mischer 5. In Fig. 4a ist eine Situation dargestellt, in der die im Mischer 5 befindliche Biomasse 8 den Kanal 20 verschließt, so dass das Biogas 9 daran gehindert ist, über die Auslassöffnung 12 abzuströmen. Durch die fortgesetzte Gasbildung im Reaktor wächst die Dicke des Gaspolsters in der Gashaube 3 stetig an, der Pegel der Biomasse 8 wird unter die Einlässe 10 abgesenkt und im Mischer 5 wird die Biomasse 8 - wie in Fig. 4b und 4c gezeigt - über den Kanal 20 und die Auslassöffnung 12 aus dem Mischer 5 verdrängt und über die Förderleitung 23 in den Gasseparator 6 gefördert. In Folge kann Biogas 9 in verstärktem Maße abströmen, wodurch das Gaspolster in der Gashaube 3 abnimmt, der Pegel der flotierenden Biomasse 8 ansteigt und - wie in Fig. 4d dargestellt - erneut Biomassse 8 in den Mischer 5 strömt.

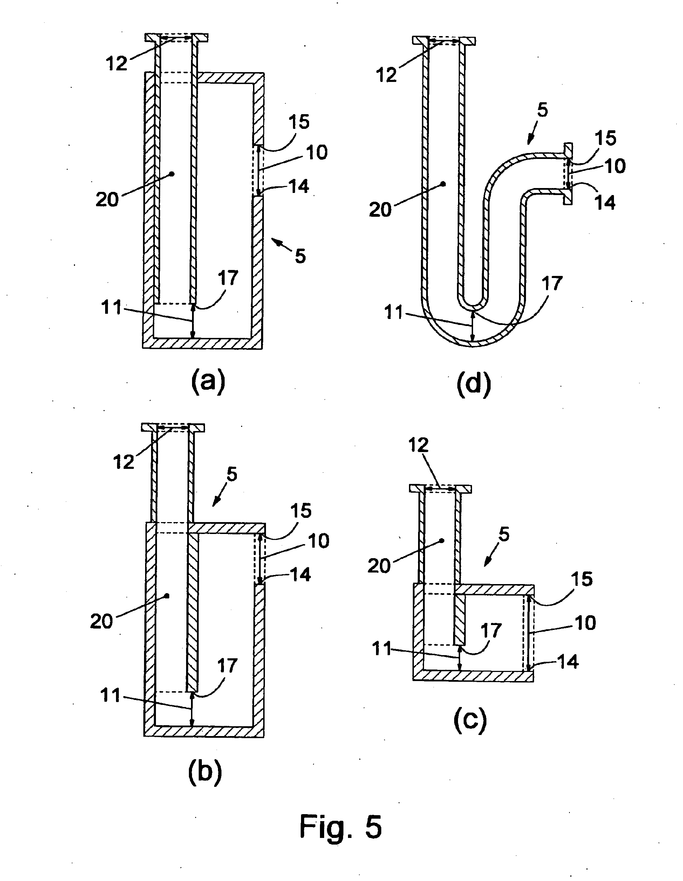

Fig. 5a bis 5d zeigen weitere Ausführungsformen von erfindungsgemäßen Mischern 5, bei denen die Einlässe 10 untere und obere Begrenzungen 14, 15 und die Durchführungen 11 obere Begrenzungen 17 aufweisen und die Begrenzungen 17 der Durchführungen 11 tiefer als die oberen Begrenzungen 15 der Einlässe 10 angeordnet sind. Die Kanäle 20 sind beispielsweise jeweils rohrförmig und verlaufen geradlinig vertikal nach oben. Bei den Weiterbildungen gemäß den Fig. 5a, 5b und 5d sind die Mischer 5 so gestaltet, dass die oberen Begrenzungen 17 der Durchführungen 11 tiefer als die unteren Begrenzungen 14 der Einlässe 10 angeordnet sind. Fig. 5d illustriert einen Mischer 5, der als U-förmiges Rohr ausgestaltet ist.

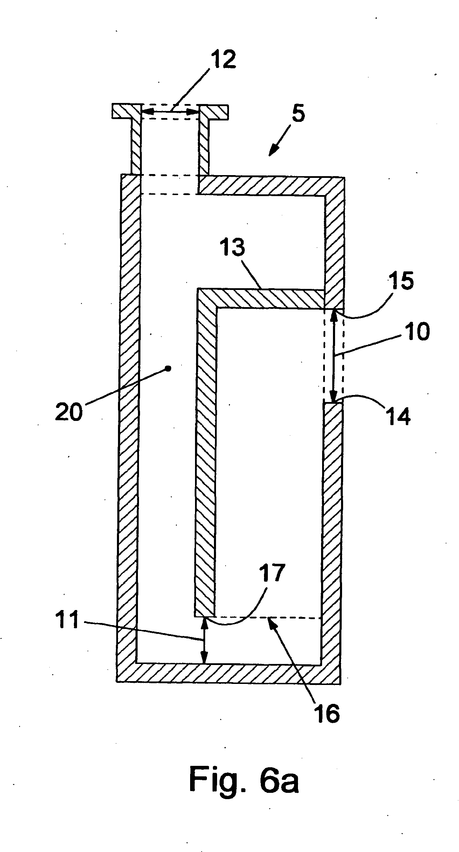

Fig. 6a zeigt eine bevorzugte Ausführungsform der Erfindung, bei der die Mischer 5 auf ihrer Innenseite mit einem oder mehreren Rückhaltekästen 13 ausgestattet sind. Die Rückhaltekästen 13 umschließen die Einlässe 10 und weisen an ihrer Unterseite Öffnungen 16 auf, die tiefer als die Einlässe 10 angeordnet sind. Durch die Öffnungen 16 und die Durchführungen 11 gelangt das Biogemenge in die Kanäle 20.

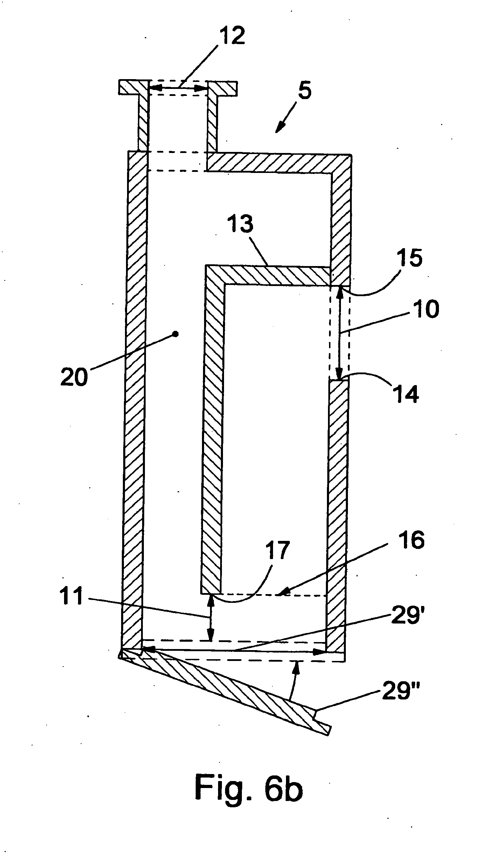

Fig. 6b illustriert ein weitere, auf die einfache Wartung und Reinigung der Mischer 5 und der Förderleitung 23 gerichtete Ausführungsform der Erfindung. In dieser Ausführungsform sind die Mischer 5 an ihrer Unterseite mit einer Wartungsöffnung 29' und einem Deckel 29" ausgestattet, wobei der Deckel 29" die Wartungsöffnung 29' gasdicht verschließt.

Fig. 7 veranschaulicht eine weitere vorteilhafte Ausführungsform der Erfindung, bei der der Mischer 5 innerhalb des Reaktortanks 1 angeordnet und mit einer nach unten weisenden Öffnung 28 versehen ist. Wie in Fig. 7 gezeigt, ist der Mischer 5 mit in zwei (oder mehreren) horizontalen Ebenen angeordneten Gashauben 3 verbunden. Alternativ hierzu können die Gashauben 3 jeweils mit separaten Mischern 5 der in Fig. 7 gezeigten Bauform versehen sein.

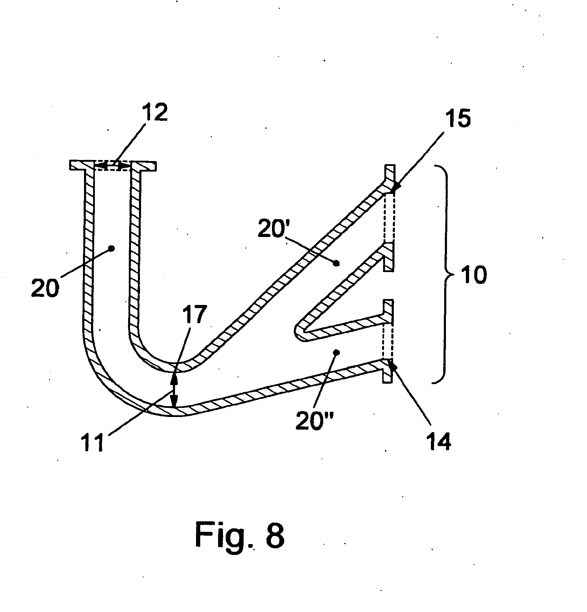

In Fig. 8 ist eine weitere erfindungsgemäße Ausgestaltung der Mischer 5 dargestellt, bei der die Einlässe 10 einen ersten Einlasskanal 20' für Biogas und einen zweiten Einlasskanal 20" für Biomasse umfassen. Die Einlasskanäle 20', 20" sind horizontal angeordnet oder in Richtung der Durchführung 11 abwärts geneigt. Der erste Einlasskanal 20' ist oberhalb von dem zweiten Einlasskanal 20" angeordnet und die obere Begrenzung 17 der Durchführung 11 liegt unterhalb der unteren Begrenzung 14 des zweiten Einlasskanals.

## Patentansprüche

1. Verfahren zur anaeroben Behandlung von Abwasser mittels eines anaeroben Aufström-Schlammbettes UASB (Upflow Anaerobic Sludge Blanket) in einem Reaktor, in dem ein Teil der als Schlamm oder Pelletschlämmung vorliegenden Biomasse (8) im Kreislauf geführt wird, durch Flotations-Trennung von Reaktorwasser (7), Biomasse (8) und Biogas (9) in einem ersten Abscheider (33), Vermischen von Biogas (9) und Biomasse (8) zu einem Gemenge nach dem Austritt aus dem ersten Abscheider (33), Trennen von Reaktorwasser (7) und Biogas (9) in einem zweiten Abscheider (35) vertikal über dem ersten Abscheider (33), Fördern des Biogemenges und des im zweiten Abscheider (35) abgetrennten Biogases (9) zu einem Gasseparator (6), Abscheiden des Biogases im Gasseparator (6) unter Freisetzung der Biomasse (8), und Rückführen der freigesetzten Biomasse (8) bis unterhalb des Schlammbettes UASB.

2. Verfahren nach Anspruch 1, **dadurch gekennzeichnet, dass** Biogas und Biomasse hydrostatisch gemischt und/oder strömungsdynamisch miteinander verwirbelt werden.

3. Verfahren nach Anspruch 1, **dadurch gekennzeichnet, dass** das Biogemenge hydrostatisch und/oder strömungsdynamisch gefördert wird.

4. Verfahren nach Anspruch 1, **dadurch gekennzeichnet, dass** die rückgeführte Biomasse mit Abwasser vermischt wird.

5. Verfahren nach Anspruch 1, **dadurch gekennzeichnet, dass** der Anteil von rückgeführter zu gesamter Biomasse im Reaktor pro Tag größer 0,1 • d⁻¹, insbesondere größer 2 • d⁻¹ und besonders bevorzugt größer 10 • d⁻¹ ist.

6. Reaktor zur anaeroben Behandlung von Abwasser mittels eines anaeroben Aufstföm-Schlammbettes (Upflow Anaerobic Sludge Blanket = UASB), umfassend einen Reaktortank (1), eine Zufuhrleitung (22) für Abwasser in einen Abwassermischer (2), einen ersten und mindestens einen zweiten Abscheider (33, 35) zur Flotations-Trennung von Reaktorwasser (7), Biomasse (8) und Biogas (9) wobei der zweite Abscheider (35) vertikal über dem ersten Abscheider (33) angeordnet ist, jeder Abscheider (33, 35) eine oder mehrere Gashauben (3, 4) aufweist und die Gashauben (3) des ersten Abscheiders (33) mit einem oder mehreren Mischern (5) zum Vermischen von Biomasse (8) und Biogas (9) zu einem Biogemenge verbunden sind und die Mischer (5) und Gashauben (4) des zweiten Abscheiders (35) über eine Förderleitung (23) an einen Gasseparator (6) zum Trennen von Biomasse (8) und Biogas (9) angeschlossen sind, eine Rücklaufleitung (24) für Biomasse (8) vom Gasseparator (6) in den Reaktor, eine Ablaufleittung (25) für Reaktorwasser aus dem Readtortank (1) und eine Abströmleitung (26) des Gasseparators (6) für Biogas (9).

7. Reaktor nach Anspruch 6, **dadurch gekennzeichnet, dass** die Mischer (5) mittels separater Verbindungsleitungen oder mittels Sammelleitungen an die Förderleitung (23) angeschlossen sind.

8. Reaktor nach Anspruch 6, **dadurch gekennzeichnet, dass** der Querschnitt der Förderleitung (23) um einen Faktor 1 : 60 bis 1 : 400 kleiner als der Querschnitt des Reaktortanks (1) ist.

9. Reaktor nach Anspruch 6, **dadurch gekennzeichnet, dass** die Förderleitung (23) teilweise oder vollständig außerhalb des Reaktortanks (1) angeordnet ist.

10. Reaktor nach Anspruch 6, **dadurch gekennzeichnet, dass** die Mischer (5) Hohlkörper sind mit einem oder mehreren Einlässen (10) für Biomasse (8) und Biogas (9), einer oder mehreren Durchführungen (11) und einer oder mehreren Auslassöffnungen (12), wobei die Einlässe (10) untere und obere Begrenzungen (14, 15) und die Durchführungen (11) obere Begrenzungen (17) aufweisen und die Begrenzungen (17) tiefer als die Begrenzungen (15) und tiefer als die Auslassöffnungen (12) angeordnet sind.

11. Reaktor nach Anspruch 10, **dadurch gekennzeichnet, dass** die oberen Begrenzungen (17) der Durchführungen (11) tiefer als die unteren Begrenzungen (14) der Einlässe (10) angeordnet sind.

12. Reaktor nach Anspruch 11, **dadurch gekennzeichnet, dass** jeder Mischer (5) auf seiner Innenseite mit einem oder mehreren Rückhaltekästen (13) ausgestattet ist, dass die Rückhaltekästen (13) die Einlässe (10) umschließen, dass die Unterseiten der Rückhaltekästen (13) Öffnungen (16) aufweisen und dass die Öffnungen (16) tiefer als die unteren Begrenzungen (14) der Einlässe (10) und tiefer als die Auslassöffnungen (12) angeordnet sind.

13. Reaktor nach Anspruch 11, **dadurch gekennzeichnet, dass** die Einlässe (10) einen ersten Einlasskanal (20') für Biogas und einen zweiten Einlasskanal (20") für Biomasse umfassen, dass die Einlasskanäle (20', 20") horizontal angeordnet oder in Richtung der Durchführung (11) abwärts geneigt sind und dass der erste Einlasskanal (20') über dem zweiten Einlasskanal (20") angeordnet ist.

14. Reaktor nach den Ansprüchen 10 bis 13, **dadurch gekennzeichnet, dass** die Mischer (5) mit Wartungsöfnungen (29') und Deckeln (29") ausgestattet sind, wobei die Deckel (29") die Wartungsöffnungen (29') gasdicht verschließen.

15. Reaktor nach den Ansprüchen 10 bis 14, **dadurch gekennzeichnet, dass** die Mischer (5) teilweise oder vollständig außerhalb des Reaktortanks (1) angeordnet sind.

16. Reaktor nach den Ansprüchen 10 bis 14, **dadurch gekennzeichnet, dass** die Mischer (5) innerhalb des Reaktortanks (1) angeordnet sind.

17. Reaktor nach Anspruch 16, **dadurch gekennzeichnet, dass** die Mischer (5) eine oder mehrere nach unten weisende Öffnungen (28) aufweisen.

18. Reaktor nach Anspruch 6, **dadurch gekennzeichnet, dass** die Gashauben (3, 4) Hohlkörper mit einer polygonalen oder halbkreisartig gekrümmten Hüllwand in Form eines umgekehrten V oder umgekehrten U mit einer nach unten weisenden Öffnung (18) sind.

19. Reaktor nach Anspruch 18, **dadurch gekennzeichnet, dass** die Gashauben (3) durch zwei ebene Stirnwände begrenzt sind, wobei mindestens eine Stirnwand eine oder mehrere Durchtrittsöffnungen (19) für Biomasse (8) und Biogas (9) aufweist.

20. Reaktor nach Anspruch 19, **dadurch gekennzeichnet, dass** die Durchtrittsöffnungen (19) im oberen Bereich der Stirnwand unterhalb des Firstes der Gashauben (3) angeordnet sind.

21. Reaktor nach Anspruch 18, **dadurch gekennzeichnet, dass** die Gashauben (3) durch ebene Stirnwände begrenzt sind, wobei die Mischer (5) mindestens eine der stirnseitigen Begrenzungen der Gashauben bilden.

22. Reaktor nach Anspruch 18, **dadurch gekennzeichnet, dass** die Gashauben (4) durch ebene Stirnwände begrenzt sind, dass mindestens eine Stirnwand Durchtrittsöffnungen (21) für Biogas (9) aufweist und dass die Durchtrittsöffnungen (21) über die Förderleitung (23) mit dem Gasseparator (6) verbunden sind, wobei die Durchtrittsöffnungen (21) über separate Verbindungsleitungen oder über Sammelleitungen an die Förderleitung (23) angeschlossen sind.

23. Reaktor nach Anspruch 22, **dadurch gekennzeichnet, dass** die Durchtrittsöffnungen (21) im oberen Bereich der Stirnwand unterhalb des Firstes der Gashauben (4) angeordnet sind.

24. Reaktor nach Anspruch 18, **dadurch gekennzeichnet, dass** die Gashauben (3, 4) in den Abscheidern (33, 35) in zwei oder mehreren übereinander liegenden horizontalen Ebenen angeordnet sind, dass die Gashauben (3, 4) einer Ebene parallel und voneinander beabstandet angeordnet sind, dass zwischen benachbarten Gashauben (3, 4) einer Ebene ein Spalt für den Durchtritt von Reaktorwasser (7) vorhanden ist und dass in jedem der Abscheider (33, 35) die Reihen der Gashauben (3, 4) in übereinander liegenden Ebenen derart zueinander versetzt sind, dass die vertikalen Projektionen der Öffnungen (18) der Gashauben (3, 4) eine geschlossene Fläche bilden, die den Innenquerschnitt des Reaktortanks (1) teilweise oder vollständig abdeckt.

25. Reaktor nach Anspruch 6, **dadurch gekennzeichnet, dass** der Gasseparator (6) im Reaktortank (1) oder außerhalb des Reaktortanks (1) und oberhalb des obersten Abscheiders (35) angeordnet ist, dass der Gasseparator (6) über die Rück- und Zufuhrleitung (24, 22) mit dem Abwassermischer (2) verbunden ist, und dass der Gasseparator (6) zur Ableitung von Biogas (9) mit der Abströmleitung (26) verbunden ist.

26. Reaktor nach Anspruch 25, **dadurch gekennzeichnet, dass** die Abströmleitung (26) mit einem Durchflußregelventil (27) ausgerüstet ist.

## Claims

1. Method for anaerobic treatment of wastewater by means of an upflow anaerobic sludge blanket UASB in a reactor, in which some of the biomass (8) present as sludge or pellet slurry is circulated, by flotation-separation of reactor water (7), biomass (8) and biogas (9) in a first separator (33), mixing of biogas (9) and biomass (8) to form a mixture after the exit from the first separator (33), separation of reactor water (7) and biogas (9) in a second separator (35) vertically above the first separator (33), conveyance of the biomixture and of the biogas (9) separated in the second separator (35) to a gas separator (6), separation of the biogas in the gas separator (6) with release of the biomass (8), and feedback of the released biomass (8) until below the sludge blanket UASB.

2. Method according to Claim 1, **characterized in that** biogas and biomass are mixed hydrostatically and/or are swirled together flow-dynamically.

3. Method according to Claim 1, **characterized in that** the biomixture is conveyed hydrostatically and/or flow-dynamically.

4. Method according to Claim 1, **characterized in that** the fed-back biomass is mixed with wastewater.

5. Method according to Claim 1, **characterized in that** the proportion of fed-back biomass to total biomass in the reactor per day is greater than 0.1 · d⁻¹, in particular greater than 2 · d⁻¹, and more preferably greater than 10 · d⁻¹.

6. Reactor for anaerobic treatment of wastewater by means of an upflow anaerobic sludge blanket (UASB), said reactor
comprising a reactor tank (1), a feed line (22) for wastewater in a wastewater mixer (2), a first and at least one second separator (33, 35) for flotation-separation of reactor water (7), biomass (8) and biogas (9), the second separator (35) being arranged vertically above the first separator (33), each separator (33, 35) having one or more gas hoods (3, 4) and the gas hoods (3) of the first separator (33) being connected to one or more mixers (5) for mixing biomass (8) and biogas (9) to form a biomixture, and the mixers (5) and gas hoods (4) of the second separator (35) being joined via a conveyor line (23) to a gas separator (6) for separating biomass (8) and biogas (9), said reactor further comprising a return line (24) for biomass (8) from the gas separator (6) into the reactor, an outlet line (25) for reactor water from the reactor tank (1) and an outflow line (26) of the gas separator (6) for biogas (9).

7. Reactor according to Claim 6, **characterized in that** the mixers (5) are joined to the conveyor line (23) by means of separate connection lines or by means of collecting lines.

8. Reactor according to Claim 6, **characterized in that** the cross-section of the conveyor line (23) is smaller than the cross-section of the reactor tank (1) by a factor of 1:60 to 1:400.

9. Reactor according to Claim 6, **characterized in that** the conveyor line (23) is arranged outside the reactor tank (1), either in part or completely.

10. Reactor according to Claim 6, **characterized in that** the mixers (5) are hollow bodies with one or more inlets (10) for biomass (8) and biogas (9), one or more ducts (11) and one or more outlet openings (12), the inlets (10) having lower and upper limits (14, 15) and the ducts (11) having upper limits (17) and the limits (17) being
arranged lower than the limits (15) and lower than the outlet openings (12).

11. Reactor according to Claim 10, **characterized in that** the upper limits (17) of the ducts (11) are arranged lower than the lower limits (14) of the inlets (10).

12. Reactor according to Claim 11, **characterized in that** each mixer (5) is equipped on its inner side with one or more retaining boxes (13), **in that** the retaining boxes (13) surround the inlets (10), **in that** the undersides of the retaining boxes (13) have openings (16), and **in that** the openings (16) are arranged lower than the lower limits (14) of the inlets (10) and lower than the outlet openings (12).

13. Reactor according to Claim 11, **characterized in that** the inlets (10) comprise a first inlet channel (20') for biogas and a second inlet channel (20") for biomass, **in that** the inlet channels (20', 20'') are arranged horizontally or are inclined downwardly in the direction of the duct (11), and **in that** the first inlet channel (20') is arranged above the second inlet channel (20'').

14. Reactor according to Claims 10 to 13, **characterized in that** the mixers (5) are equipped with maintenance openings (29') and covers (29''), the covers (29'') closing the maintenance openings (29') in a gastight manner.

15. Reactor according to Claims 10 to 14, **characterized in that** the mixers (5) are arranged outside the reactor tank (1), either in part or completely.

16. Reactor according to Claims 10 to 14, **characterized in that** the mixers (5) are arranged inside the reactor tank (1).

17. Reactor according to Claim 16, **characterized in that** the mixers (5) have one or more downwardly pointing openings (28).

18. Reactor according to Claim 6, **characterized in that** the gas hoods (3, 4) are hollow bodies having a polygonal casing wall or a casing wall curved in a semi-circular-like manner, in the form of an inverted V or inverted U with a downwardly pointing opening (18).

19. Reactor according to Claim 18, **characterized in that** the gas hoods (3) are delimited by two planar end walls, at least one end wall having one or more through-openings (19) for biomass (8) and biogas (9).

20. Reactor according to Claim 19, **characterized in that** the through-openings (19) are arranged in the upper region of the end wall below the apex of the gas hoods (3).

21. Reactor according to Claim 18, **characterized in that** the gas hoods (3) are delimited by planar end walls, the mixers (5) forming at least one of the end limits of the gas hoods.

22. Reactor according to Claim 18, **characterized in that** the gas hoods (4) are delimited by planar end walls, **in that** at least one end wall has through-openings (21) for biogas (9), and **in that** the through-openings (21) are connected via the conveyor line (23) to the gas separator (6), the through-openings (21) being joined to the conveyor line (23) via separate connection lines or via collecting lines.

23. Reactor according to Claim 22, **characterized in that** the through-openings (21) are arranged in the upper region of the end wall below the apex of the gas hoods (4).

24. Reactor according to Claim 18, **characterized in that** the gas hoods (3, 4) in the separators (33, 35) are arranged
in two or more horizontal planes arranged one above the other, **in that** the gas hoods (3, 4) in a plane are arranged in a mutually parallel and spaced manner, **in that** a gap for the passage of reactor water (7) is provided between adjacent gas hoods (3, 4) in a plane, and **in that**, in each of the separators (33, 35), the rows of the gas hoods (3, 4) are offset from one another in planes arranged one above the other, in such a way that the vertical projections of the openings (18) of the gas hoods (3, 4) form a closed area, which covers the inner cross-section of the reactor tank (1) either in part or completely.

25. Reactor according to Claim 6, **characterized in that** the gas separator (6) is arranged in the reactor tank (1) or outside the reactor tank (1) and above the uppermost separator (35), **in that** the gas separator (6) is connected via the return and feed line (24, 22) to the wastewater mixer (2), and **in that** the gas separator (6) for removing biogas (9) is connected to the outflow line (26).

26. Reactor according to Claim 25, **characterized in that** the outflow line (26) is equipped with a flow control valve (27).

## Revendications

1. Procédé de traitement anaérobie d'eaux usées à l'aide d'un réacteur anaérobie à lit de boues à flux ascendant UASB (Upflow Anaerobic Sludge Blanket) dans un réacteur dans lequel une partie de la biomasse (8) présente sous forme de boue ou d'une décantation de boues granulaires est amenée dans la circulation, par séparation par flottation de l'eau du réacteur (7), de la biomasse (8) et du biogaz (9) dans un premier séparateur (33), mélange du biogaz (9) et de la biomasse (8) pour former un mélange après la sortie du premier séparateur (33), séparation de l'eau du réacteur (7) du biogaz (9) dans un deuxième séparateur (35) disposé verticalement au-dessus du premier séparateur (33), alimentation du biomélange et du biogaz (9) séparé dans le deuxième séparateur (35) à un séparateur de gaz (6), séparation du biogaz dans le séparateur de gaz (6) en libérant la biomasse (8) et réinjection de la biomasse (8) libérée juste en dessous du lit de boues UASB.

2. Procédé selon la revendication 1, **caractérisé en ce que** le biogaz et la biomasse sont mélangés de manière hydrostatique et/ou tourbillonnés l'un avec l'autre par la dynamique d'écoulement.

3. Procédé selon la revendication 1, **caractérisé en ce que** le biomélange est acheminé de manière hydrostatique et/ou par la dynamique d'écoulement.

4. Procédé selon la revendication 1, **caractérisé en ce que** la biomasse réinjectée est mélangée avec des eaux usées.

5. Procédé selon la revendication 1, **caractérisé en ce que** la proportion de la biomasse réinjectée par rapport à la biomasse totale dans le réacteur par jour est supérieure à 0,1.d⁻¹, en particulier supérieure à 2.d⁻¹ et, tout particulièrement, supérieure à 10.d⁻¹.

6. Réacteur pour le traitement anaérobie d'eaux usées à l'aide d'un réacteur anaérobie à lit de boues à flux ascendant UASB (Upflow Anaerobic Sludge Blanket) comprenant une cuve de réacteur (1), une conduite d'alimentation (22)
pour les eaux usées dans un mélangeur d'eaux usées (2), un premier et au moins un deuxième séparateurs (33, 35) pour la séparation par flottation de l'eau du réacteur (7), de la biomasse (8) et du biogaz (9), le deuxième séparateur (35) étant disposé verticalement au-dessus du premier séparateur (33), chaque séparateur (33, 35) comprenant un ou plusieurs collecteurs de gaz (3, 4) et le collecteur de gaz (3) du premier séparateur (33) étant relié avec un ou plusieurs mélangeurs (5) pour le mélange de la biomasse (8) et du biogaz (9) en un biomélange et les mélangeurs (5) et les collecteurs de gaz (4) du deuxième séparateur (35) étant reliés par l'intermédiaire d'une conduite d'alimentation (23) à un séparateur de gaz (6) pour la séparation de la biomasse (8) et du biogaz (9), une conduite de réinjection (24) pour la biomasse (8) du séparateur de gaz (6) dans le réacteur, une conduite d'écoulement (25) pour l'eau du réacteur hors de la cuve du réacteur (1) et une conduite d'évacuation (26) du séparateur de gaz (6) pour le biogaz (9).

7. Réacteur selon la revendication 6, **caractérisé en ce que** les mélangeurs (5) sont raccordés à l'aide de conduites de raccordement séparées ou à l'aide de conduites collectrices à la conduite d'alimentation (23).

8. Réacteur selon la revendication 6, **caractérisé en ce que** la section de la conduite d'alimentation (23) est inférieure d'un facteur 1:60 à 1:400 à la section de la cuve du réacteur (1).

9. Réacteur selon la revendication 6, **caractérisé en ce que** la conduite d'alimentation (23) est disposée en tout ou en partie à l'extérieur de la cuve du réacteur (1).

10. Réacteur selon la revendication 6, **caractérisé en ce que** les mélangeurs (5) sont des corps creux avec une ou plusieurs admissions (10) pour la biomasse (8) et le biogaz (9), un ou plusieurs passages traversants (11) et une ou plusieurs ouvertures d'évacuation (12), les admissions (10) présentant des limitations supérieures et inférieures (14, 15) et les passages traversants (11) présentant des limitations supérieures (17) et les limitations (17) étant disposées plus profondément que les limitations (15) et plus profondément que les ouvertures
d'évacuation (12).

11. Réacteur selon la revendication 10, **caractérisé en ce que** les limitations supérieures (17) des passages traversants (11) sont disposées plus profondément que les limitations inférieures (14) des admissions (10).

12. Réacteur selon la revendication 11, **caractérisé en ce que** chaque mélangeur (5) est doté sur sa face intérieure d'un ou plusieurs boîtiers de retenue (13), que les boîtiers de retenue (13) renferment les admissions (10), que les faces inférieures des boîtiers de retenue (13) présentent des ouvertures (16) et que les ouvertures (16) sont disposées plus profondément que les limitations inférieures (14) des admissions (10) et plus profondément que les ouvertures d'évacuation (12).

13. Réacteur selon la revendication 11, **caractérisé en ce que** les admissions (10) comprennent un premier canal d'admission (20') pour le biogaz et un deuxième canal d'admission (20") pour la biomasse, que les canaux d'admission (20', 20") sont disposés horizontalement ou inclinés vers le bas dans la direction du passage traversant (11) et que le premier canal d'admission (20') est disposé au-dessus du deuxième canal d'admission (20").

14. Réacteur selon l'une des revendications 10 à 13, **caractérisé en ce que** les mélangeurs (5) sont dotés d'orifices de maintenance (29') et de couvercles (29"), les couvercles (29") fermant les orifices de maintenance (29') de manière étanche aux gaz.

15. Réacteur selon l'une des revendications 10 à 14, **caractérisé en ce que** les mélangeurs (5) sont disposés en tout ou en partie en dehors de la cuve du réacteur (1).

16. Réacteur selon l'une des revendications 10 à 14, **caractérisé en ce que** les mélangeurs (5) sont disposés à l'intérieur de la cuve du réacteur (1).

17. Réacteur selon la revendication 16, **caractérisé en ce que** les mélangeurs (5) présentent une ou plusieurs ouvertures (28) tournées vers le bas.

18. Réacteur selon la revendication 6, **caractérisé en ce que** les collecteurs de gaz (3, 4) sont des corps creux avec une paroi enveloppante polygonale ou en demi-cercle sous la forme d'un V renversé ou d'un U renversé avec une ouverture (18) tournée vers le bas.

19. Réacteur selon la revendication 18, **caractérisé en ce que** les collecteurs de gaz (3) sont délimités par deux faces d'extrémité planes, au moins une face d'extrémité présentant une ou plusieurs ouvertures traversantes (19) pour la biomasse (8) et le biogaz (9).

20. Réacteur selon la revendication 19, **caractérisé en ce que** les ouvertures traversantes (19) sont disposées dans la région supérieure de la face d'extrémité en-dessous du plafond des collecteurs de gaz (3).

21. Réacteur selon la revendication 18, **caractérisé en ce que** les collecteurs de gaz (3) sont délimités par des faces d'extrémité planes, les mélangeurs (5) formant au moins l'une des limitations sur la face d'extrémité des collecteurs de gaz.

22. Réacteur selon la revendication 18, **caractérisé en ce que** les collecteurs de gaz (4) sont délimités par des faces d'extrémité planes, qu'au moins une face d'extrémité présente des ouvertures traversantes (21) pour le biogaz (9) et que les ouvertures traversantes (21) sont reliées par l'intermédiaire de la conduite d'alimentation (23) avec le séparateur de gaz (6), les ouvertures 30 traversantes (21) étant raccordées par l'intermédiaire de conduites de raccordement séparé ou par l'intermédiaire de conduites collectrices à la conduite d'alimentation (23).

23. Réacteur selon la revendication 22, **caractérisé en ce que** les ouvertures traversantes (21) sont disposées dans la région supérieure de la face d'extrémité en dessous du plafond des collecteurs de gaz (4).

24. Réacteur selon la revendication 18, **caractérisé en ce que** les collecteurs de gaz (3, 4) dans les séparateurs (33, 35) sont disposés dans deux plans horizontaux superposés ou plus, que les collecteurs de gaz (3, 4) d'un plan sont disposés parallèlement et espacés l'une de l'autre, qu'une fente est présente pour le passage de l'eau du réacteur (7) entre deux collecteurs de gaz (3, 4) voisins d'un niveau et que, dans chacun des séparateurs (33, 35), les rangées des collecteurs de gaz (3, 4) sont déplacées l'une vers l'autre dans des niveaux superposés de telle sorte que les projections verticales des orifices (18) dans les collecteurs de gaz (3, 4) forment une surface fermée qui recouvre en tout ou en partie la section interne de la cuve du réacteur (1).

25. Réacteur selon la revendication 6, **caractérisé en ce que** le séparateur de gaz (6) est disposé dans la cuve du réacteur (1) ou à l'extérieur de la cuve du réacteur (1) et au-dessus du séparateur (35) supérieur, le séparateur de gaz (6) est relié par l'intermédiaire de la conduite de réinjection et d'alimentation (24,22) avec le mélangeur d'eaux usées (2) et le séparateur de gaz (6) est relié pour la déviation du biogaz (9) avec la conduite d'évacuation (26).

26. Réacteur selon la revendication 25, **caractérisé en ce que** la conduite d'évacuation (26) est équipée d'une soupape régulatrice du débit (27).
